# EUROPEAN PATENT APPLICATION

(11) **EP 2 281 816 A1**
(43) Date of publication of application: **09.02.2011**
(21) Application number: 09754438.1
(22) Date of filing: 27.05.2009
(51) Int. Cl.: C07D 213/70, A01N 43/40, A01N 55/02, A61P 3/00, A61K 8/58, A61Q 5/02, A61Q 7/00, C09D 5/16, C09D 7/12

(54) **NEW CRYSTALLINE PYRITHIONE/ZINC OXIDE COMPLEX AND PHYSIOLOGIC/ANTIBIOTIC ACTIVE COMPOSITE CONTAINING THE SAME**

(30) Priority: 30.05.2008 JP 2008167909; 01.06.2008 JP 2008167923
(71) Applicant: YHS Ltd., Sakai-shi, Osaka 5900114 (JP)
(72) Inventor: HIDAKA, Yasuhiro, Sakai-shi Osaka 590-0114 (JP)
(74) Representative: von Kreisler Selting Werner
(86) International application number: PCT/JP2009/002336
(87) International publication number: WO 2009/144929

(57) **Abstract**

Although zinc pyrithione is known as an excellent antidandruff component, there is a demand for an effort to reduce the blending amount without lowering the effects from the viewpoint of environmental pollution. Also, there is a demand for developing a technique that enables combining zinc pyrithione with cuprous oxide to be used as an antifouling component for a ship-bottom paint. Moreover, also as an antiseptic and anti-mold component for polymer materials, further enhancement of the antimicrobial activity of zinc pyrithione has been demanded.

It has been elucidated that a new composite of the present invention obtained by treating an aqueous suspension or an aqueous paste containing zinc pyrithione or a zinc pyrithione/zinc oxide complex compound and ultrafine zinc oxide having an average particle diameter of 0.01 to 0.15 µm at pH 8 to 12 exerts an excellent physiologic/antibiotic activity such as an anti-dandruff and hair restoration effect and an underwater antifouling, antiseptic and anti-mold effect. Thus, the above-described problems have been solved.

## Description

### TECHNICAL FIELD

The present invention relates to a new crystalline pyrithione/zinc oxide composite. In more detail, the present invention relates to a new crystalline pyrithione/zinc oxide composite obtained by treating an aqueous suspension or an aqueous paste containing zinc pyrithione or a zinc pyrithione/zinc oxide complex compound and ultrafine zinc oxide at pH 8 or higher and a physiologic/antibiotic active composition containing the same. More specifically, the present invention relates to an antidandruff and hair restoration composition, an underwater antifouling composition, and an antiseptic and anti-mold composition.

### BACKGROUND ART

An aqueous suspension such as a shampoo obtained by blending zinc pyrithione and zinc oxide is disclosed in Japanese Patent Laid-Open No. 52-92881 (Patent Document 1). The aqueous suspension is characterized by containing zinc oxide as a stabilizer in order to stabilize zinc pyrithione, which is decomposed by light or iron ions. However, there is absolutely no description regarding use of ultrafine zinc oxide having an average particle diameter of 0.15 µm or smaller.

A hair restoration shampoo containing zinc oxide, boric acid, and starch is also known as disclosed in U.S. Patent No. 6033653 (Patent Document 2). However, there is no description regarding ultrafine zinc oxide having an average particle diameter of 0.15 µm or smaller, and further, the shampoo requires troublesome steps including rubbing it into the scalp and keeping it there for 25 minutes, then rinsing it off.

Also, WO 01/00151 (Patent Document 3) discloses a shampoo containing a composition having zinc pyrithione and metal ions including zinc ions derived from zinc oxide. Although it is suggested that an antimicrobial activity against Malassezia, which is a dandruff-causing fungus, is enhanced by the composition and thus the antidandruff effect is enhanced, there is no description regarding ultrafine zinc oxide having an average particle diameter of 0.15 µm or smaller.

National Publication of International Patent Application No. 2006-515330 (Patent Document 4) discloses that a combination of zinc pyrithione and a particulate zinc substance successfully and dramatically can improve an anti-dandruff effect of a topical composition, listing particulate zinc oxide as an example of particulate zinc substance. This document discloses zinc oxide that could presumably be ultrafine zinc oxide having an average particle diameter of 0.06 µm as one kind of the particulate zinc oxide, and describes an example of shampoo in which the above zinc oxide is formulated with zinc pyrithione. However, because the shampoo liquid is adjusted to have a weakly acidic pH, the composite of the present invention, which only forms at pH 8 or higher, is not formed.

National Publication of International Patent Application No. 2003-522734 (Patent Document 5) discloses that a combination of zinc pyrithione and zinc oxide enhances an antimicrobial and anti-mold effect of zinc pyrithione. However, there is also no description regarding ultrafine zinc oxide having an average particle diameter of 0.15 µm or smaller in this document.

National Publication of International Patent Application No. 2002-521339 (Patent Document 6) discloses a particle having a core/shell structure, where zinc oxide is the core and zinc pyrithione is the shell. However, the amount of zinc pyrithione attached to zinc oxide is approximately only 10 wt. % or less with respect to zinc oxide, and therefore the antimicrobial activity of zinc pyrithione cannot be fully exerted.

Japanese Patent No. 4185526 (Patent Document 7) discloses a technique for producing a zinc pyrithione/zinc oxide complex compound from sodium pyrithione, excess zinc sulfate, and sodium hydroxide. However, generation of by-products of large zinc oxide particles is unavoidable with this production method. That is, because zinc oxide generated as a by-product has a large particle size, it will be present as free zinc oxide without forming a composite with a zinc pyrithione/zinc oxide complex compound. As a result, when mass-producing a zinc pyrithione/zinc oxide complex compound, zinc oxide, for its large specific gravity, deposits in the lower layer in a reaction batch, making it difficult to achieve homogenous quality within a lot. In an aqueous suspension preparation, there is a drawback that the content is separated into two layers.

Japanese Patent Laid-Open No. 2006-335757 (Patent Document 8) discloses a technique for atomizing (i) a zinc pyrithione/zinc oxide complex compound or a composition composed of at least one kind of (i) a zinc pyrithione/zinc oxide complex compound, (ii) zinc pyrithione, and (iii) zinc oxide and a technique for combining ultrafine zinc oxide therewith with an aim to improve dispersion stability. However, because preparation and atomization of the aforementioned complex compound and composition and combining of ultrafine zinc oxide therewith are not carried out at pH 8 or higher, the composite of the present invention is not produced. Further, there is absolutely no data implying formation of the composite of the present invention in the specification.

Japanese Patent Laid-Open No. 6-134227 (Patent Document 9) discloses a method for producing an antimicrobial filter including dispersing ultrafine zinc oxide having an average particle diameter of 0.02 µm and zinc pyrithione (an average particle diameter of 0.07 µm) in an aqueous emulsion of resin; immersing a filter in the dispersion liquid thus obtained; and drying the filter thus obtained to adsorb antimicrobial components to the surface thereof. However, because the dispersion liquid containing zinc pyrithione and ultrafine zinc oxide is treated at neutral pH, the composite of the present invention is not formed, and it goes without saying that there is no description indicative of production of the composite of the present invention.

Patent Document 1: Japanese Patent Laid-Open No. 52-92881
Patent Document 2: U.S. Patent No. 6033653
Patent Document 3: WO 01/00151
Patent Document 4: National Publication of International Patent Application No. 2006-515330
Patent Document 5: National Publication of International Patent Application No. 2003-522734
Patent Document 6: National Publication of International Patent Application No. 2002-521339
Patent Document 7: Japanese Patent No. 4185526
Patent Document 8: Japanese Patent Laid-Open No. 2006-335757
Patent Document 9: Japanese Patent Laid-Open No. 6-134227

### DISCLOSURE OF THE INVENTION

### PROBLEMS TO BE SOLVED BY THE INVENTION

As described above, zinc pyrithione is formulated in a shampoo as an antidandruff agent. However, because it has a strong irritant action on the ocular mucosa, and also for the environmental hygiene reasons, an effort has been continuously made to minimize the blending amount of zinc pyrithione without reducing the antidandruff effect.
Also, in order to achieve a hair restoration effect and a hair loss-inhibiting effect, after rubbing a shampoo for hair restoration containing zinc oxide into the scalp, it needs to be kept on the scalp as it is for approximately 25 minutes. Accordingly, a technique with which the effects of the shampoo can also be obtained in an ordinary usage, namely rinsing the hair right after shampooing, has been demanded.

Zinc pyrithione is blended in a ship-bottom paint composition as an antifouling agent. However, when zinc oxide and zinc pyrithione are formulated together, there is a risk that the paint might turn into a gel during storage; therefore, a technique for preventing the gelation has been demanded. Also, while a composition composed of zinc pyrithione and zinc oxide, which is used as an antiseptic and anti-mold agent, has an improved non-staining property compared to zinc pyrithione alone, the composition has not been considered to have a sufficient effect.

### SOLUTIONS TO THE PROBLEMS

The present inventor has found that a homogenous crystalline composite previously undescribed in the literature is produced by treating an aqueous dispersion liquid or an aqueous paste containing zinc pyrithione or a zinc pyrithione/zinc oxide complex compound and ultrafine zinc oxide having an average particle diameter of 0.01 to 0.15 µm at pH 8 to 12, particularly pH 8 to 9, and the composite thus produced has a strong antidandruff and hair restoration effect and an antibiotic activity, thereby completing the present invention.
That is, the present inventions are:
(1) a new crystalline zinc pyrithione/zinc oxide composite obtained by treating an aqueous suspension or an aqueous paste comprising zinc pyrithione or zinc pyrithione/ amorphous zinc oxide complex compound and ultrafine zinc oxide having an average particle diameter of 0.01 to 0.15 µm at pH 8 to 12, the zinc pyrithione or the zinc pyrithione/ amorphous zinc oxide complex compound being represented by the general formula (I):

   xZnO·ZnPy₂ (I)

   wherein x represents 0 or a positive number satisfying 0≤x≤1 and Py represents a 2-pyridylthio-N-oxide group;
(2) the new crystalline zinc pyrithione/zinc oxide composite according to (1), which is obtained by treating the aqueous suspension or the aqueous paste at pH 8 to 9;
(3) the new crystalline zinc pyrithione/zinc oxide composite according to (1) or(2), wherein an amount of the ultrafine zinc oxide having an average particle diameter of 0.01 to 0.15 µm is 1 to 50 parts by weight per 100 parts by weight of the zinc pyrithione or the zinc pyrithione/zinc oxide complex compound represented by the general formula (I) according (1) in the aqueous suspension or the aqueous paste;
(4) a physiologic/antibiotic active composition comprising the new crystalline zinc pyrithione/zinc oxide composite according to any of (1) to (3);
(5) the physiologic/antibiotic active composition according to (4), which is an anti-dandruff and hair restoration agent;
(6) the physiologic/antibiotic active composition according to (5), further comprising an azole antifungal agent;
(7) the physiologic/antibiotic active composition according to (5) or (6), which is a shampoo;
(8) the physiologic/antibiotic active composition according to (4), which is an underwater antifouling agent;
(9) the physiologic/antibiotic active composition according to (4) or (8), which is a ship-bottom paint further comprising cuprous oxide;
(10) the physiologic/antibiotic active composition according (4), which is an antiseptic and anti-mold agent; and
(11) the physiologic/antibiotic active composition according to (10), which is an aqueous antiseptic and anti-mold suspension further comprising an isothiazolone antiseptic and anti-mold agent.

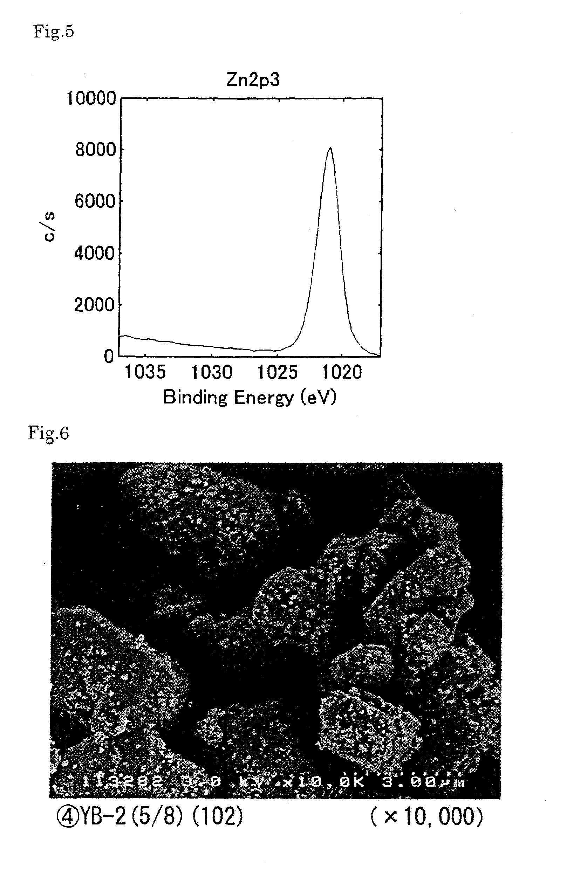

The invention achieved by the present inventor as described in Japanese Patent No. 4185526 and the present invention differ in that the product disclosed in the above patent is composed of a mixture of a zinc pyrithione/zinc oxide complex compound and zinc oxide having an average particle diameter of approximately 5 µm generated as a by-product, whereas the product of the present invention is a crystalline composite obtained by treating zinc pyrithione or a zinc pyrithione/zinc oxide complex compound and ultrafine zinc oxide having an average particle diameter of 0.01 to 0.15 µm at pH 8 to 12, and filtering and drying the resulting product as needed.
"A zinc pyrithione/zinc oxide complex compound" herein means a complex compound wherein the zinc pyrithione is physicochemically compounded with amorphous zinc oxide, and it is obtained by extracting a reaction mixture by chloroform to separate and remove crystalline zinc oxide. The chloroform extract thus obtained has a greater content of zinc than zinc pyrithione; however, because it is amorphous, the peak of diffraction angle of zinc oxide cannot be observed by the X-ray diffraction analysis.
On the other hand, "a composite of zinc pyrithione or a zinc pyrithione/zinc oxide complex compound and ultrafine zinc oxide" means a composite which is composed of zinc pyrithione or a zinc pyrithione/zinc oxide complex compound and ultrafine zinc oxide having an average particle diameter of 0.01 to 0.15 µm physicochemically bound thereto. The composite can be confirmed by downward shift of the binding energy of Zn2p3 and the presence of single peak by the X-ray photoelectron spectroscopy (XPS). It can also be confirmed by an external appearance in which visible ultrafine zinc oxide particles adhere to the surface of zinc pyrithione particles in a scanning electron microscope (SEM) photograph as shown in Fig. 6. Furthermore, the composite differs from the complex compound in that the composite shows the peak of diffraction angle of zinc oxide by the X-ray diffraction analysis.

Although the invention disclosed in Japanese Patent No. 4185526 and the present invention are both commonly produced in an alkaline condition and having zinc pyrithione and zinc oxide as components of a product, they are produced as different substances by different production methods. One of the reasons accounting for this is considered to be the size of a zinc oxide particle. That is, an average particle diameter of zinc oxide produced as a by-product by the production method described in the above patent is approximately several µm, which is not so different from the particle diameter of zinc pyrithione. Thus, crystallizable zinc oxide produced as a by-product cannot adhere to a crystal of zinc pyrithione, and therefore it is considered not to form a composite with zinc pyrithione/ amorphous zinc oxide complex compound, which is simultaneously produced.

Further, although the inventions disclosed in Patent Documents 4, 8, and 9 and the present invention all commonly use zinc pyrithione and ultrafine zinc oxide as raw materials, there is a difference that compositions are produced according to the above patents, while a composite is produced by the present invention. The reason accounting for this is presumed as follows; while zinc oxide is not activated at an acidic to neutral region, it is activated at an alkaline region, whereby an interaction with a zinc complex portion in zinc pyrithione is enhanced and a physicochemical bond is formed.
It is evident that a crystalline composite is formed when zinc pyrithione and ultrafine zinc oxide are alkaline-treated because the peak exothermic temperature of a product obtained by the treatment has risen by approximately 15°C in a differential thermal analysis (DTA) (Example 1), and also, as described above, each peak representing a binding energy of zinc oxide and zinc pyrithione Zn2p3 has disappeared and the binding energy has shifted downward as found by the XPS. Meanwhile, in the XPS of a mixture of zinc pyrithione and ultrafine zinc oxide, peaks representing the spectrum peak of each of zinc pyrithione and ultrafine zinc oxide were confirmed.

The composite of the present invention is considered to be a new composite previously undescribed in the literature. There is no description suggesting formation of the composite in any of the above-described Patent Documents 4, 8, and 9 describing compositions of zinc pyrithione and ultrafine zinc oxide. Also, even considering the pH of aqueous suspensions or aqueous preparations containing the compositions, formation of the composite is unthinkable. That is, normally a pH of a shampoo is adjusted to 6.5 to 7.0 so as not to cause irritation in the eye mucosa, and the pH is never adjusted to higher than 8.
Further, the difference between the new crystalline zinc pyrithione/zinc oxide composite of the present invention and a mixture of zinc pyrithione and ultrafine zinc oxide, namely a composition, is notably manifested in the antimicrobial activity and hair restoration effect. The composite of the present invention has twice the antimicrobial activity on Malassezia fungus and twice or more the hair restoration effect in comparison with a composition of zinc pyrithione and ultrafine zinc oxide.
Furthermore, while the composite of the present invention exhibits, in comparison with a mixture of zinc pyrithione and ultrafine zinc oxide, twice to four times the antimicrobial effect on E.coli and pseudomonas aeruginosa, which are the major contaminants of emulsion paints, it has an excellent gelation-preventing effect in a ship-bottom paint containing cuprous oxide during storage.

The new crystalline zinc pyrithione/zinc oxide composite of the present invention is obtained by stirring an aqueous suspension or an aqueous paste containing zinc pyrithione or zinc pyrithione/ amorphous zinc oxide complex compound represented by the above-described general formula (I) and ultrafine zinc oxide having an average particle diameter of 0.01 to 0.15 µm at pH 8 to 12, preferably at pH 8 to 10, particularly preferably at pH 8 to 9, and drying the resulting solid.
When stirring the aqueous suspension or the aqueous paste, heating is not particularly required; however, it can be heated to 20 to 95°C. Stirring time is normally five minutes to four hours, preferably ten minutes to three hours. Preferable alkaline agents used to adjust pH of the aqueous suspension or the aqueous paste include sodium hydroxide, potassium hydroxide, sodium carbonate, and ammonia.

The composite obtained by treating an aqueous suspension or an aqueous paste containing zinc pyrithione or a zinc pyrithione/zinc oxide complex compound and ultrafine zinc oxide having an average particle diameter of 0.01 to 0.15 µm, preferably 0.02 to 0.09 µm at pH 8 to 12 can be used as it is in the form of aqueous suspension or aqueous paste, without collecting by filtration and drying.

When zinc pyrithione and ultrafine zinc oxide having an average particle diameter of 0.01 to 0.15 µm are treated at pH 9 or higher, particularly at pH 9.5 or higher, zinc pyrithione is converted to an alkali metal salt of pyrithione such as sodium pyrithione. Further, at pH 9.5 or higher, particularly at pH 11 or higher, zinc oxide transiently becomes zincate, and then converted to amorphous zinc oxide, whereby the zinc pyrithione/zinc oxide complex compound disclosed in Japanese Patent No. 4185526 is produced. It is to be noted that when normal or ordinary grade zinc oxide having an average particle diameter of 0.6 µm is used as zinc oxide in place of ultrafine zinc oxide having an average particle diameter of 0.01 to 0.15 µm, the zinc pyrithione/zinc oxide complex compound disclosed in Japanese Patent No. 4185526 is still produced at a similar pH condition.
The white powder thus obtained is extracted in chloroform, and production of the zinc pyrithione/zinc oxide complex compound is confirmed when the zinc content of the chloroform extract is found to exceed the zinc content of zinc pyrithione. Accordingly, in that case, the zinc pyrithione/zinc oxide composite of the present invention contains a composite composed of a zinc pyrithione/zinc oxide complex compound and ultrafine zinc oxide having an average particle diameter of 0.01 to 0.15 µm.

As zinc pyrithione to be used as a raw material of the present invention, a commercial powder product produced for a ship-bottom paint can be used. Also, a 48 wt. % aqueous suspension commercially available for a shampoo can be used. The above-described zinc pyrithione products are supplied by Arch Chemicals, Inc., Kolon Life Science Inc., API Corporation, and the like. A zinc pyrithione/zinc oxide complex compound can be produced following the method described in Japanese Patent No. 4185526, which is the prior invention made by the present inventor.

As ultrafine zinc oxide having an average particle diameter of 0.01 to 0.15 µm used as the other raw material of the present invention, a commercial product developed as an ultraviolet screening agent for cosmetics can be used. The above-described ultrafine zinc oxide product having an average particle diameter of 0.01 to 0.15 µm is obtainable from the market, and it is supplied by, for example, Tayca Corporation, Sakai Chemical Industry Co., Ltd., HakusuiTech Co., Ltd., and Sumitomo Cement Co., Ltd.

The composite of the present invention is composed of 1 to 50 parts by weight, preferably 2 to 25 parts by weight of ultrafine zinc oxide having an average particle diameter of 0.01 to 0.15 µm per 100 parts by weight of the above-described zinc pyrithione or zinc pyrithione/zinc oxide complex compound. When the content of the ultrafine zinc oxide is less than 1 wt. %, a sufficient combinational effect of the ultrafine zinc oxide and zinc pyrithione or the zinc pyrithione/zinc oxide complex compound cannot be obtained. Further, when the content of the ultrafine zinc oxide is 50 wt. % or more, the content of the ultrafine zinc oxide in the composite will not increase.

The composite of the present invention is formulated in a shampoo in an amount of 0.2 to 2.0 wt. %, preferably 0.5 to 1.0 wt. %, in order to prevent dandruff. Further, the composite of the present invention is formulated in a shampoo in an amount of 0.2 to 2.0 wt. %, preferably 0.5 to 1.0 wt. %, in order to add a hair restoration effect. Accordingly, two purposes, i.e., dandruff prevention and hair restoration, can be achieved simultaneously with addition of the composite of the present invention to a shampoo in an amount of 0.2 to 2.0 wt.%, preferably 0.5 to 1.0 wt.%.

An azole antifungal agent such as ketoconazole, miconazole nitrate, clotrimazole, itraconazole, climbazole, thioconazole, and fluconazole has a strong antimicrobial activity against Malassezia, which is a fungus associated with dandruff production, and it is formulated in a commercial shampoo as an anti-dandruff agent. However, an azole antifungal agent is expensive, and there is a concern of side effects such as rash, itching, and dryness on the scalp. There is another concern that zinc pyrithione is irritative to the eye mucosa. However, by combining the physiologic/antibiotic active composite of the present invention with an azole antifungal agent, a synergistic antimicrobial effect is exerted on Malassezia. Further, addition of a combination of the physiologic/antibiotic active composite of the present invention and an azole antifungal agent to an anti-dandruff shampoo generates an economic effect, and further, can alleviate the side effects associated with each of them. When the composite of the present invention is formulated in a shampoo in combination with an azole antifungal agent, the composite of the present invention is formulated in the shampoo in an amount of 0.1 to 1.0 wt. %, preferably 0.2 to 0.7 wt. %. Similarly, the azole antifungal agent is formulated in the shampoo in amount of 0.1 to 1.5 wt. %, preferably 0.2 to 1.0 wt. %.

Further, the composite of the present invention can be mixed in combination with antidandruff, hair restoring active component such as zinc pyrithione, piroctone olamine, salicylic acid, dipotassium glycyrrhizate, isopropyl methylphenol, pyridoxine hydrochloride, α-tocopherol, calcium pantothenate, nicotinamide, biotin, glyceryl pentadecanoate, carpronium chloride, menthol, hinokitiol, a ginkgo extract, peppermint oil, cantharis, and minoxidil.

A cleansing agent to be simultaneously employed when the composite of the present invention is formulated in a shampoo is preferably an anionic or nonionic surfactant. Examples of the anionic surfactant include a sodium, ammonium, monoethanolamine, or diethanolamine salt of lauryl sulfate, a sodium, potassium, ammonium, monoethanolamine, or diethanolamine salt of lauryl ether sulfate, and palm oil fatty acid monoglyceride sodium sulfate. Examples of the nonionic surfactant include a condensation product of nonylphenol and ethylene oxide, a condensation product of palm oil alcohol and ethylene oxide, dodecyldimethylamine oxide, and dodecyl dimethyl phosphine oxide. Further, purified water, a fragrance, a colorant, a viscosity-enhancer, an antiseptic, a dispersion-stabilizer, a pH adjusting agent, a foam-forming agent, a pearlescent agent, and the like are appropriately used.

While zinc pyrithione is known as an antifouling component against algae, it often causes gelation during storage when formulated in a ship-bottom paint composition with cuprous oxide. The reason for the above is considered as follows; copper ions are dissociated into water present in the paint, and converted into divalent copper ions. The divalent copper ions then cause a metal substitution reaction with zinc pyrithione, producing reactive zinc ions. Besides that the composite of the present invention exerts an effect as an antifouling component, it has an inhibitory effect on a metal substitution reaction with copper ions. The composite of the present invention is formulated in a paint for the bottom of a ship and an antifouling agent for a fishing net in an amount of 0.5 to 10.0 wt. %, preferably 1 to 5
wt. %. Also, cuprous oxide is formulated in a ship-bottom paint and an antifouling agent for a fishing net in an amount of 5 to 50 wt. %, preferably 10 to 40 wt. %.

Preferable resin components to be formulated in the ship-bottom paint and the antifouling agent for a fish-farming net simultaneously together with the composite of the present invention include an acrylic resin. Also, an antifouling component such as cuprous oxide, copper thiocyanate, a metallic copper product, zinc oxide, and zinc dithiocarbamate can be formulated. Further, a solvent, a color pigment, an extender pigment, a viscosity-adjuster, a sedimentation-preventing agent, a dripping-preventing agent, and the like are appropriately selected and used.

The composite of the present invention is used as an antiseptic and anti-mold component by blending it in an aqueous emulsion or an aqueous dispersion liquid such as an emulsion paint, an adhesive, a polymer emulsion, a drilling fluid, a coating color in an amount of 0.01 to 5 wt. %, preferably 0.1 to 2 wt. %. The composite of the present invention is supplied in the form of an aqueous suspension or aqueous paste for the above-described purposes of use. On the other hand, for a polymer material such as plastic, rubber, and fiber, it is supplied in the form of powder as an anti-mold component. The blending amount is 0.2 to 5 wt. %, preferably 0.4 to 2.5 wt. %.

The antimicrobial activity of the antibiotic active composite of the present invention against Pseudomonas and Aspergillus can be improved by using it in combination with an isothiazolone antiseptic and anti-mold component. Meanwhile, the strong skin irritancy of the isothiazolone antiseptic and anti-mold component can be alleviated. Specific examples of such isothiazolone antiseptic and anti-mold component include 2-methyl-4-isothiazolin-3-one, 2-octyl-4-isothiazolin-3-one, and 5-chloro-2-methyl-4-isothiazolin-3-one.
The antibiotic active composite of the present invention and an isothiazolone antiseptic and anti-mold component are blended in a weight ratio of 10:1 to 1:10. In the aforementioned industrial products, the above substances are used, as the anti-septic component, in a combined amount of 0.005 to 1.0 wt. %, preferably 0.01 to 0.5 wt. %, and as the anti-mold component, in a combined amount of 0.1 to 5 wt. %, preferably 0.5 to 2.5 wt. %.

### ADVANTAGES OF THE INVENTION

The physiologic/antibiotic active composite of zinc pyrithione or a zinc pyrithione/zinc oxide and ultrafine zinc oxide having an average particle diameter of 0.01 to 0.15 µm according to the present invention exhibits excellent anti-dandruff and hair restoration effects when formulated in a shampoo. Also, the antibiotic active composite of zinc pyrithione or a zinc pyrithione/zinc oxide and ultrafine zinc oxide having an average particle diameter of 0.01 to 0.15 µm according to the present invention has, when blended in a ship-bottom paint or an antifouling agent for a fish-farminging net with cuprous oxide, an inhibitory effect on gelation, which is likely to be generated when zinc pyrithione and cuprous oxide are used in combination. Further, the antibiotic active composite of the present invention exhibits a superior antimicrobial effect than does zinc pyrithione as an antiseptic and anti-mold component of an aqueous product for industrial use such as the emulsion paint and a polymer material.

### BRIEF DESCRIPTION OF THE DRAWINGS

Fig. 1 is a narrow XPS spectrum of the complex compound obtained in Example 1 (a chloroform extract);
Fig. 2 is a narrow XPS spectrum of the composite obtained in Example
Fig. 3 is a narrow XPS spectrum of the composite obtained in Example
Fig. 4 is a narrow XPS spectrum of ultrafine zinc oxide (manufactured by Tayca Corporation);
Fig. 5 is a narrow XPS spectrum of zinc pyrithione (manufactured by Arch Chemicals, Inc);
Fig. 6 is an SEM photograph of the composite obtained in Example 3;
Fig. 7 is an SEM photograph of zinc pyrithione (manufactured by Arch Chemicals, Inc);
Fig. 8 is an SEM photograph of ultrafine zinc oxide (manufactured by Tayca Corporation);
Fig. 9 is a temperature-corrected diagram of differential thermal analysis (DTA) of the composite obtained in Example 1. In the Figure, 1, 2, and 3 represents temperature (Temp), DTA, and thermal gravimetric analysis (TGA), respectively.
Fig. 10 is a temperature-corrected diagram of DTA of the composite obtained in Example 2. In the Figure, 1, 2, and 3 represents Temp, DTA, and TGA, respectively.
Fig. 11 is a temperature-corrected diagram of DTA of zinc pyrithione (manufactured by Arch Chemicals, Inc). In the Figure, 1, 2, and 3 represents Temp, DTA, and TGA, respectively.

### EMBODIMENT OF THE INVENTION

The present invention will be described specifically hereinbelow with reference to Examples.

### Example 1

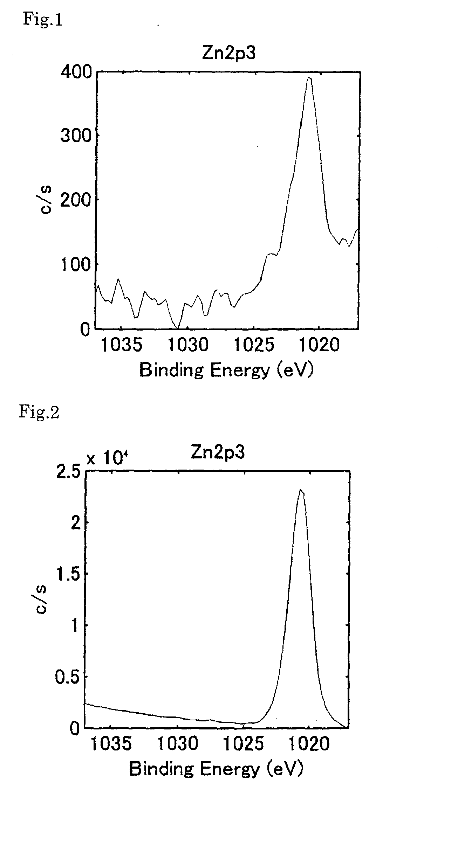

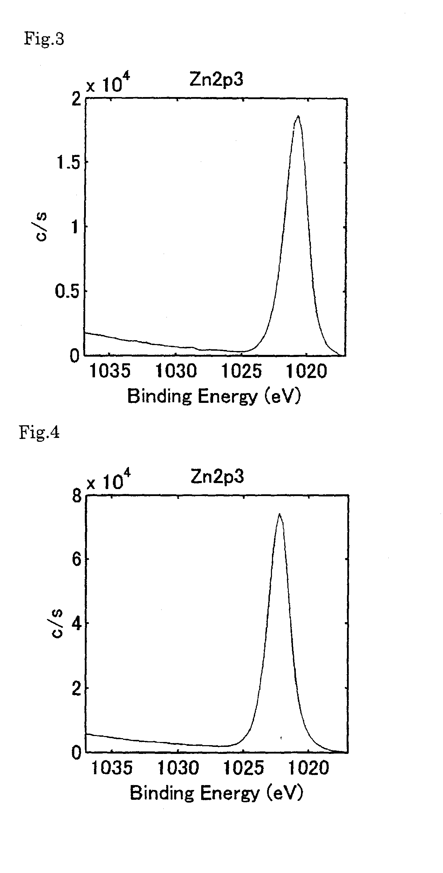

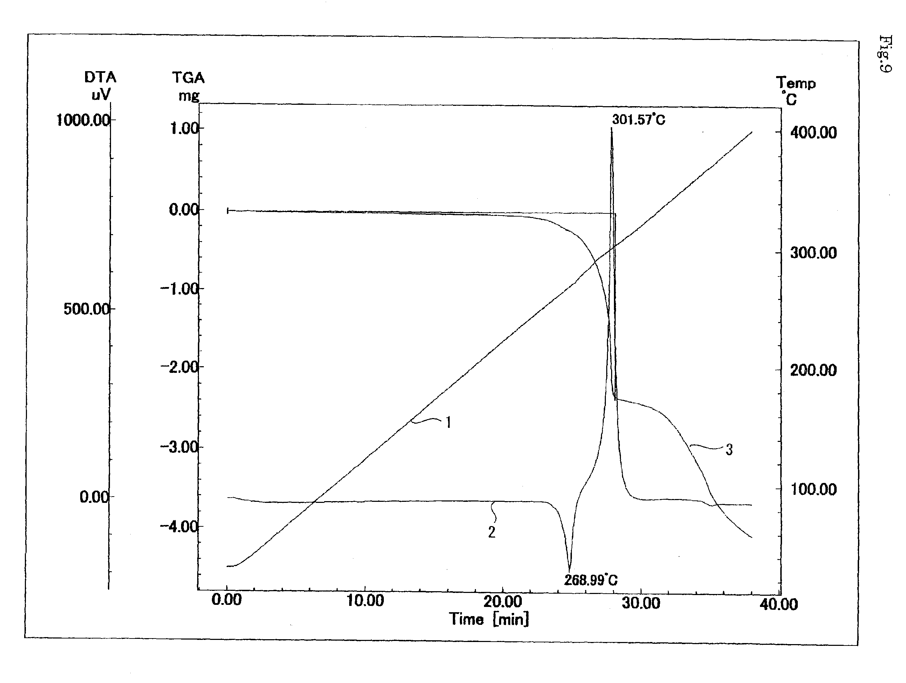

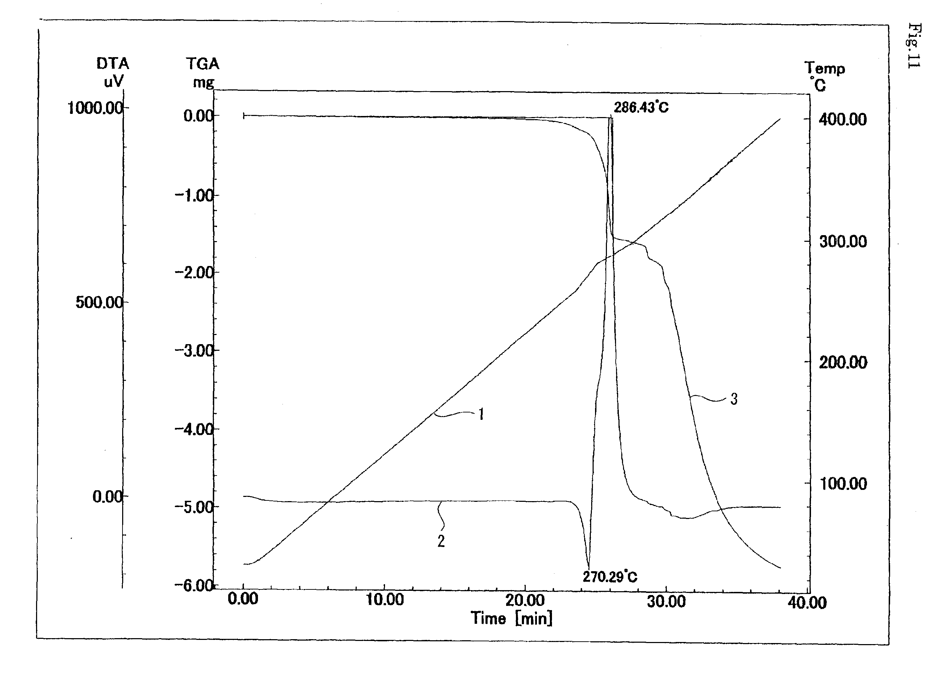

To 3500 mL of water in a 5-L flask, 276.7 g of zinc pyrithione powder (manufactured by Arch Chemicals, Inc) and 70.7 g of ultrafine zinc oxide having an average particle diameter of 0.03 µm were added. The resulting mixture was adjusted to pH 12 with a 1% aqueous sodium hydroxide solution, followed by stirring at 90°C for 10 minutes. The mixture was once cooled and then adjusted to pH 9.5 with 5% hydrochloric acid, followed by stirring for two hours while being maintained at 80°C. After cooling, the mixture was filtered through No. 2 filter paper. The moist solid left on the filter paper was put back in a container, and operations of washing with water and filtering were repeated until the filtrate became clear. The moist solid obtained by filtration was dried at 50°C for five hours to give 340.4 g of white powder. The results obtained by a thermal gravimetric analysis (heating rate: 10°C/min) performed on the white powder were shown in Fig. 9. The peak exothermic temperature was 301.6°C (after correction). Compared to raw material zinc pyrithione (Fig. 11), the peak exothermic temperature was found to have risen approximatey by 15°C.
Two 800 mg portions of the white powder thus obtained were separately added to 600 mL of chloroform in 1-L flasks. The resulting mixtures were stirred at 60°C for 60 minutes and filtered through membrane filters, followed by drying. The white powder left on the filters thus obtained (i) weighed 150 mg and 160 mg in the first and second operations, respectively, and the white powder obtained by distillation of the filtrates (ii) weighed 640 mg and 630 mg in the first and second operations, respectively.
Further, (i) and (ii) were each subjected to the X-ray fluorescence analysis. As a result, substantially only zinc was detected, while sulfur derived from zinc pyrithione was not detected in (i). Also, regarding (ii), it was found to contain 1.09 times the amount of zinc (an average value of two operations) than the zinc content of raw material zinc pyrithione based on a comparison with respect to sulfur in zinc pyrithione. That is, a composite composed of a zinc pyrithione/zinc oxide complex compound to which 9% of zinc oxide in a molar ratio with respect to zinc pyrithione is bound (x = 0.09) and ultrafine zinc oxide of x = 0.91 was obtained. It is to be noted that because the above-described chloroform extract (ii) did not exhibit a diffraction peak specific to zinc oxide as a result of the X-ray diffraction analysis, zinc oxide bound to zinc pyrithione is considered to be amorphous. Also, the results of the XPS analysis of the above chloroform extract were shown in Fig. 1. The binding energy of Zn2p3 was shifted by 0.3 eV downward compared to that of zinc pyrithione (Fig. 5). Further, a spectrum peak indicating the binding energy of ultrafine zinc oxide was found to be disappeared (Fig. 4).

### Example 2

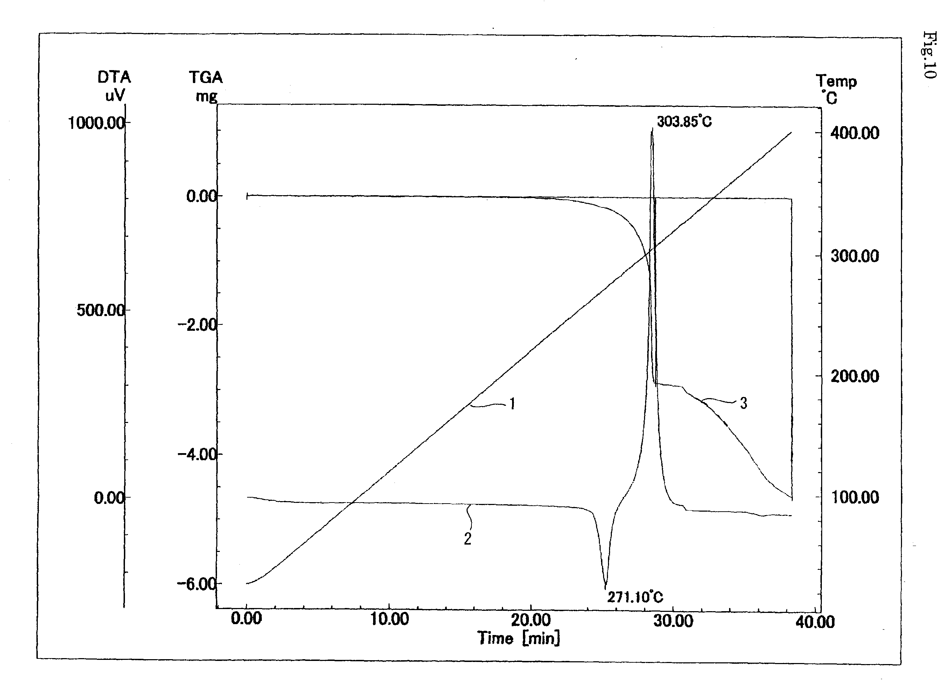

To a 200-mL beaker, 4.8 g of zinc pyrithione powder (manufactured by Kolon Life Science Inc.) and 0.6 g of ultrafine zinc oxide having an average particle diameter of 0.03 µm (manufactured by Tayca Corporation) (zinc pyrithione:ultrafine zinc oxide = 2:1 in a molar ratio) were added, to which 100 mL of distilled water was added. The resulting mixture was adjusted to pH 8.0 with a 1% aqueous sodium hydroxide solution. The aqueous slurry thus obtained was stirred at 20°C for 30 minutes, and solid was collected by filtration through No. 2 filter paper. The solid thus obtained was washed with 50 mL of distilled water twice and dried at 50°C for five hours to give 5.1 g of white powder. The results obtained by a thermal gravimetric analysis (heating rate: 10°C/min) performed on the white powder were shown in Fig. 10. The peak exothermic temperature was 303.9°C (after correction). A composite composed of zinc pyrithione and ultrafine zinc oxide, which was 0.5 with respect to zinc pyrithione in a molar ratio (x = 0.5), was obtained. Also, the results of the XPS analysis of the composite were shown in Fig. 2. Single peak spectrum of the binding energy of Zn2p3 was observed, and the binding energy of Zn2p3 was shifted by 1.5 eV and 0.5 eV downward compared to those of ultrafine zinc oxide and zinc pyrithione (Figs. 4 and 5), respectively.

### Comparative Example 1

To a 200-mL beaker, 10 g of a 48% aqueous zinc pyrithione suspension (manufactured by Kolon Life Science Inc.) and 0.6 g of ultrafine zinc oxide having an average particle diameter of 0.03 µm (manufactured by Tayca Corporation) (zinc pyrithione:ultrafine zinc oxide = 2:1 in a molar ratio) were added, to which 100 mL of distilled water was added. The pH of the resulting mixture was 7. The mixture was stirred at 20°C for 30 minutes, and decantation was performed twice using 50 mL of distilled water. Subsequently, the cake thus obtained was dried at 50°C for five hours to give 5.0 g of white powder. As a result of a thermal gravimetric analysis performed on the white powder in the same manner as in Example 2, the peak exothermic temperature was 294.5°C (after correction).

### Comparative Example 2

Using normal grade zinc oxide having an average particle diameter of 0.6 µm (manufactured by HakusuiTech Co., Ltd.) in place of the ultrafine zinc oxide of Example 2, 5.2 g of white powder was prepared in the same manner as in Example 2. As a result of a thermal gravimetric analysis performed on the white powder, the peak exothermic temperature was 299.4°C (after correction).

### Comparative Example 3

Using normal grade zinc oxide having an average particle diameter of 0.6 µm (manufactured by HakusuiTech Co., Ltd.) in place of the ultrafine zinc oxide of Comparative Example 1, 5.1 g of white powder was prepared in the same manner as in Comparative Example 1. As a result of a thermal gravimetric analysis performed on the white powder, the peak exothermic temperature was 290.0°C (after correction).

### Example 3

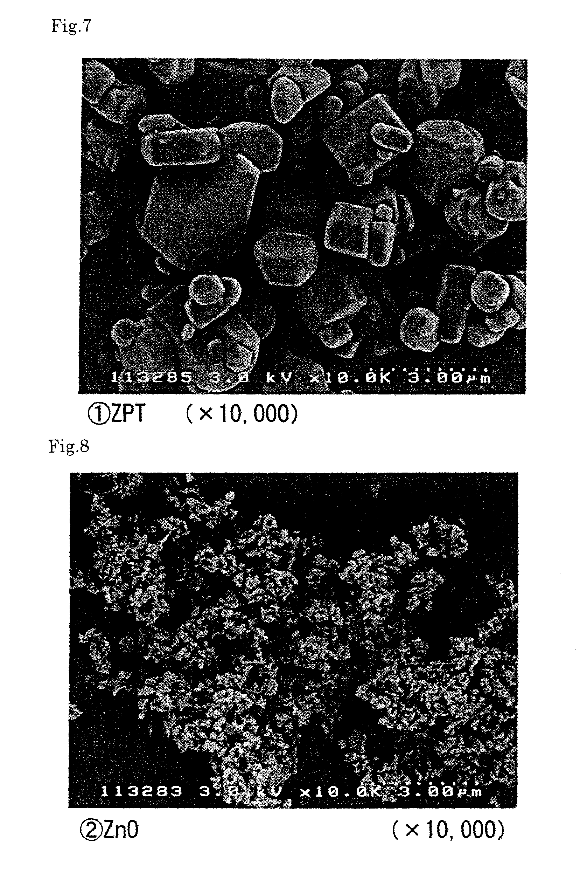

To 250 mL of water in a 500-mL flask, 200 g of zinc pyrithione powder (manufactured by API Corporation) and 1.3 g of ultrafine zinc oxide having an average particle diameter of 0.03 µm (manufactured by Tayca Corporation) (zinc pyrithione:ultrafine zinc oxide = 4:1 in a molar ratio) were added. The resulting mixture was adjusted to pH 12 with a 1% aqueous sodium hydroxide solution, followed by stirring at 90°C for 10 minutes. The mixture was once cooled and then adjusted to pH 9.5 with 5% hydrochloric acid, followed by stirring for two hours while being maintained at 80°C. After cooling, the mixture was filtered through No. 2 filter paper. Similarly to Example 1, operations of washing with water and filtering were repeated until the filtrate became clear. The solid thus obtained was dried at 50°C for five hours to give 20.8 g of white powder. As a result of a thermal gravimetric analysis (heating rate: 10°C/min) performed on the white powder, the peak exothermic temperature was 294.7°C (after correction). Also, an SEM photograph of the white powder was shown in Fig. 6. Ultrafine zinc oxide adhering to the surface of zinc pyrithione particles was observed (Fig. 7). The above fine zinc oxide is considered to be a relatively large particle constituting a part of ultrafine zinc oxide particles (Fig. 8). It is presumed that a part of the rest of the ultrafine zinc oxide particles was converted to an amorphous form, and further another part of the rest was an ultrafine particle that cannot be clearly recognized by visual observation.
Subsequently, 800 mg of the white powder thus obtained was subjected to chloroform extraction in the same manner as in Example 1. Then, the residue after distillation was found to contain 1.04 times the amount of zinc compared to zinc pyrithione as found by the X-ray fluorescence analysis. That is, a composite composed of a zinc pyrithione/zinc oxide complex compound to which 4% of zinc oxide with respect to zinc pyrithione in a molar ratio is bound (x = 0.04) and ultrafine zinc oxide of x = 0.21 was obtained. The results of the XPS analysis of the composite were shown in Fig. 3. Single peak spectrum of the binding energy of Zn2p3 was observed, and the binding energy of Zn2p3 was shifted by 1.4 eV and 0.4 eV downward compared to those of ultrafine zinc oxide and zinc pyrithione (Figs. 4 and 5), respectively.

### Example 4

Following a method described in Example 3 of Japanese Patent No. 4185526, a synthesis was carried out in a preparation ratio of sodium pyrithione: zinc sulfate heptahydrate: sodium hydroxide = 1:5/8:1/4 (molar ratio) (the pH after reaction was 9.5). Precipitated white powder was filtrated, and the moist solid left on filter paper was put back in a container. Operations of washing with water and filtering were repeated to obtain a cake, which was dried and pulverized to give 13.8 g of white powder (A). Since the yield of the white powder was 99%, it is understood that the white powder contained 13.0 g of zinc pyrithione component and 0.8 g of zinc oxide component. Also, the white powder thus obtained was extracted in chloroform, and the zinc content of the extract (residue after distillation) was quantitated by the X-ray fluorescence analysis. As a result, it was found that it contained 1.20 times the amount of zinc with respect to zinc pyrithione. That is, a complex compound composed of a zinc pyrithione/zinc oxide complex compound to which 20% of zinc oxide was bound (x = 0.20) was obtained. Thus, it is understood that zinc oxide was produced as a by-product in an amount equivalent to 0.05 in a molar ratio.
To 80 mL of distilled water in a 200 mL-beaker, 3.4 g of the white powder thus obtained (A) and 0.6 g of ultrafine zinc oxide (an average particle diameter of 0.03 µm) (manufactured by Tayca Corporation) were added (pH 8). Subsequently, operations were carried out in the same manner as in Example 2, whereby 3.8 g of white powder (B) was obtained. As a result of a thermal gravimetric analysis performed on the white powder, the peak exothermic temperature was 307.4°C (after correction).

### Example 5

Antimicrobial tests of the white powder obtained in Example 2 and Comparative Examples 1 to 3 against E.coli and Staphylococcus aureus were carried out. Also, the results obtained as to the white powder (B) and white powder (C) obtained by adding ultrafine zinc oxide having an average particle diameter of 0.03 µm (manufactured by Tayca Corporation) to the chloroform extract of the white powder of Example 4 (A) so that the amount of a zinc oxide component was equal to that of (B) were shown together in Table 1.

### Antimicrobial test

### Test method:

Sample suspensions were prepared by dispersing 80 mg of each sample in sterilized purified water. Two-fold dilution series of the above liquids were prepared using a 0.01 wt. % aqueous solution of Tween 80. From each liquid, 1 mL was dispensed into petri dishes, to which 9 mL of heat-sterilized test media were added, whereby plates were prepared.

### Test bacterial strain:

E.coli (Escherichia coli NBRC 3972)
Staphylococcus aureus (Staphylococcus aureus NMRC 12732)

### Culture condition:

Normal agar (NBA) media, 32°C, five days

**[Table 1]**

| Minimum Inhibitory Concentration (MIC, µg/ml) | | |
|---|---|---|
| Sample | E.coli | Staphylococcus aureus |
| White powder of Example 2 | 6.25 | 3.125 |
| White powder of Comparative Example 1 | 25 | 6.25 |
| White powder of Comparative Example 2 | 12.5 | 6.25 |
| White powder of Comparative Example 3 | 50 | 25 |
| White powder of Example 4 (B) | 6.25 | 3.125 |
| White powder (C) | 12.5 | 6.25 |

From the results of the above table, the composite of zinc pyrithione and ultrafine zinc oxide exhibited a superior antimicrobial activity than did the mixture of zinc pyrithione and ultrafine zinc oxide or normal grade zinc oxide. Also, the composite (B) obtained by the complex compound and ultrafine zinc oxide exhibited a superior antimicrobial activity than did the mixture (C) of the complex compound and ultrafine zinc oxide.

### Example 6

Aqueous suspensions containing any two or three components of zinc pyrithione, ultrafine zinc oxide, normal grade zinc oxide, a 50% aqueous solution of 2-methyl-4-isothiazolin-3-one were treated at pH 8, and antimicrobial tests of each of the above aqueous suspensions against pseudomonas and E.coli were carried out. The results are shown in Table 2.
Sample 1: Zinc pyrithione (manufactured by API Corporation) + ultrafine zinc oxide ("FINEX-50", manufactured by Sakai Chemical Industry, Co., Ltd., average particle diameter: 0.02 µm) (mixing ratio by weight = 4:1)
Sample 2: Zinc pyrithione (manufactured by API Corporation) + normal grade zinc oxide (manufactured by Sakai Chemical Industry, Co., Ltd., average particle diameter: 0.6 µm) (mixing ratio by weight = 4:1)
Sample 3: Zinc pyrithione (manufactured by API Corporation) + ultrafine zinc oxide ("FINEX-50", manufactured by Sakai Chemical Industry, Co., Ltd., average particle diameter: 0.02 µm) + a 50% aqueous solution of 2-methyl-4-isothiazolin-3-one (manufactured by Shoei Kagaku KK.) (mixing ratio by weight = 4:1:4)
Sample 4: Zinc pyrithione (manufactured by API Corporation)
Preparation of samples: Aqueous suspensions having the above-described compositions were prepared to have a zinc pyrithione concentration of 16 mg/mL by stirring for 15 minutes under a condition of pH 8. Two-fold dilution series of the above suspensions were prepared using a 0.1 wt. % weakly alkaline aqueous solution of Tween 80 (pH 8) and then dispensed into sterilized petri dishes, to which agar media were added, whereby plates were prepared.

### Test bacterial strain:

Pseudomonas (Pseudomonas aeruginosa JCM 6119) E.coli (Escherichia coli NBRC 3972)

### Culture condition:

Normal agar media, 32°C, five days

**[Table 2]**

| Minimum Inhibitory Concentration (MIC, µg/ml) | | |
|---|---|---|
| Sample | Pseudomonas | E.coli |
| Sample 1 | 50 | 6.25 |
| Sample 2 | 200 | 12.5 |
| Sample 3 | 12.5 | 3.125 |
| Sample 4 | >400 | 25 |

From the results of Table 1 in Example 5 and the above table, sample 1 suggests formation of the composite of zinc pyrithione and ultrafine zinc oxide. That is, it is suggested that, after obtaining a powder composite of zinc pyrithione and ultrafine zinc oxide, instead of producing an aqueous composite suspension with addition of water, an aqueous composite suspension can be directly produced by treating zinc pyrithione and ultrafine zinc oxide at pH 8. Also, it is suggested that an antimicrobial activity against Pseudomonas, which is perceived as a weak point of the zinc pyrithione antiseptic, is considerably improved by combining an isothiazolone antiseptic with sample 1.

### Example 7

Antimicrobial tests of the white powder of Example 4 (B) (a composite of a zinc pyrithione/zinc oxide complex compound and ultrafine zinc oxide) and a mixture of zinc pyrithione powder ("Clean-Bio ZP", manufactured by Kolon Life Science Inc.) + ultrafine zinc oxide ("MZ-300", manufactured by Tayca Corporation, average particle diameter: 0.03 µm) (1:0.25, weight ratio) against Malassezia were carried out. The results are shown in Table 3.
Test method: In sterilized 0.1 wt. % aqueous solutions of Tween 80, 80 mg of each sample were dispersed to prepare 10 mL of test liquids. Two-fold dilution series of the test liquids were prepared using a 0.01 wt. % aqueous solution of Tween 80. From each liquid, 1 mL was dispensed into petri dishes, to each of which 9 mL of heat-sterilized agar medium solutions at 50°C were gradually added. The resulting mixtures were thoroughly mixed so that the samples became homogenous and then provided as plates.

### Test fungal strain:

Malassezia furfur NBRC 0656 (10⁶ cfu/mL)

### Test medium:

an agar medium of pH 6.0 containing, per litter thereof, 10 g of glucose, 5 g of peptone, 3 g of yeast extract, 3 g of malt extract, and 15 g of agar, to which 10 g of olive oil was added
Culture temperature: 28°C
Cultivating period: four days

**[Table 3]**

| Minimum Inhibitory Concentration (MIC, µg/ml) | | |
|---|---|---|
| | White powder of Example 4 (B) | Mixture of zinc pyrithione powder + ultrafine zinc oxide (1:0.25, weight ratio |
| Malassezia furfur | 6.25 | 12.5 |

From the results of the above table, the composite of a zinc pyrithione/zinc oxide complex compound and ultrafine zinc oxide exhibited twice the antimicrobial activity against Malassezia in comparison with the composition containing equal amounts of zinc pyrithione component and zinc oxide entirely as ultrafine zinc oxide.

### Example 8

Antimicrobial tests of the white powder of Example 4 (B), zinc pyrithione, miconazole nitrate, and ketoconazole against Malassezia were carried out. Also, a combinational effect of the white powder of Example 4 (B), miconazole nitrate, and ketoconazole was tested.

The results are shown in Table 4.
Sample 1: white powder of Example 4 (B)
Sample 2: zinc pyrithione powder (manufactured by Arch Chemicals, Inc.)
Sample 3: miconazole nitrate (reagent manufactured by Sigma-Aldrich Corporation)
Sample 4: Pulverized ketoconazole powder ("Ketosin" 200 mg/tablet, manufactured by Europharm Laboratories Co., Ltd.)
Sample 5: 1:1 mixture (weight ratio) of white powder of Example 4 (B) and miconazole nitrate (reagent manufactured by Sigma-Aldrich Corporation)
Sample 6: 1:1 mixture (weight ratio) of white powder of Example 4 (B) and pulverized ketoconazole powder ("Ketosin" 200 mg/tablet, manufactured by Europharm Laboratories Co., Ltd.)

### Test method:

In sterilized 0.1 wt. % aqueous solutions of Tween 80, 40 mg of each of samples 1 to 3, 51.6 mg of sample 4 (40 mg in pure quantity), 20 mg of each of samples 1 and 3 as sample 5, and 40 mg in pure quantity of sample 6, which is obtained by mixing 20 mg of sample 1 and 25.8 mg (20 mg in pure quantity) of sample 4 were each suspended to prepare 10 mL of test liquids. Two-fold dilution series of the test liquids were prepared using a 0.01 wt. % aqueous solution of Tween 80. From each liquid, 0.1 mL was dispensed into 0.88 mL of Sabouraud glucose agar media. After thoroughly mixing, the media were inoculated with 0.02 mL of test fungal liquids prepared at 2 × 10⁶ cfu/mL, to which 0.01 to 0.02 mL of olive oil was added. The samples thus prepared were subjected to shaking culture at 30 to 32°C for two to four days, after which the growth of the fungi was examined.

### Test fungal strain:

Malassezia furfur NBRC 0656

**[Table 4]**

| Minimum Inhibitory Concentration against Malassezia (MIC, µg/ml) | | |
|---|---|---|
| Sample | 2 days | 4 days |
| Sample 1 | 1.56 | 3.12 |
| Sample 2 | 6.25 | 6.25 |
| Sample 3 | 0.78 | 1.56 |
| Sample 4 | 0.78 | 0.78 |
| Sample 5 | 0.39 | 0.78 |
| Sample 6 | 0.78 | 0.78 |

From the results of the above table, the composite of a zinc pyrithione/zinc oxide complex compound and ultrafine zinc oxide exhibited twice the antimicrobial activity against Malassezia in comparison with zinc pyrithione.
The above results suggest that an anti-dandruff shampoo containing the composite of the present invention has a superior anti-dandruff effect than does a conventional anti-dandruff shampoo containing zinc pyrithione. The results further suggest that a combination of miconazole nitrate, ketoconazole, and the composite of the present invention has a synergistic antimicrobial activity against Malassezia.

### Example 9

A hair restoration test was carried out using six male 7-week-old Hartley guinea pigs.
Because only three sites were available for application of a sample per guinea pig, the guinea pigs were divided into two groups for the test (I and II). A day before dividing, the hair in the back was shaved by an electric shaver and then removed by hair removal cream. Three sites (A, B, and C) having a size of 5 cm² (2 cm × 2.5 cm) centering on the back spinal line were prepared as administration sites. To these administration sites, 0.5 mL of the below-described samples was applied once a day, and wearing rubber gloves that were changed for each sample, the sample was lightly rubbed with fingers. Five minutes after application, the samples were lightly rubbed off with tap water-moistened cotton. The above operations were continuously performed for four days, and three days thereafter, the condition of hair growth was observed. The same treatments were further continued for five days, and three days thereafter, the condition of hair growth was observed. Subsequently, the hair grown in the application sites was cut off with scissors and measured for weight. The results are shown in Table 5.
Sample 1-1: 0.6 wt. % aqueous suspension of white powder of Example 4 (B) (0.1 wt. % Tween 80)
Sample 1-2: aqueous suspension of 0.6 wt. % white powder of Example 4 (B) + 3.0 wt. % ultrafine zinc oxide ("MZ-300", manufactured by Tayca Corporation, an average particle diameter of 0.03 µm) (0.1 wt. % Tween 80)
Negative control 1 (blank): 0.1 wt. % aqueous solution of Tween 80

**[Table 5]**

| Hair weight (mg) | | | | | | |
|---|---|---|---|---|---|---|
| Animal No. | Sample 1-1 | | Sample 1-2 | | Negative control 1 | |
| | I | II | I | II | I | II |
| 1 | 87.3 | 63.4 | 89.3 | 74.2 | 49.1 | 40.5 |
| 2 | 62.6 | 102.8 | 72.0 | 94.7 | 37.9 | 60.1 |
| 3 | 90.5 | 74.2 | 101.7 | 58.2 | 59.9 | 61.1 |
| Average value | 80.1 | | 81.7 | | 51.4 | |
| Standard deviation | 16.1 | | 16.3 | | 10.5 | |
| Standard error | 6.6 | | 6.7 | | 4.3 | |
| Rate of hair increase vs. negative control | 56% | | 59% | | - | |

### Comparative Example 4

A hair restoration test was carried out using six male 7-week-old Hartley guinea pigs.
Because only three sites were available for application of a sample per guinea pig, the guinea pigs were divided into two groups for the test (I and II). To the areas of 5 cm² (2 cm × 2.5 cm) in the back where the hair was shaven and removed by hair removal cream, 0.5 mL of the below-described samples was applied once a day, and after thoroughly rubbing it for five minutes, the samples were rinsed off with purified water. The above operations were continuously performed for four days, and three days thereafter, the condition of hair growth was observed and the weight of the hair in the sample-applied sites was measured. The results are shown in Table 6.
Sample 2-1: (I and II) 1.0 wt. % aqueous suspension of a mixture of a zinc pyrithione/zinc oxide complex compound synthesized following Example 3 of the specification of Patent No. 4185526 and zinc oxide produced as a by-product (0.1 wt. % Tween 80)
Sample 2-2: (I and II) 2.0 wt. % aqueous suspension of a 48% aqueous dispersion liquid of zinc pyrithione ("CleanBio-Zinc", manufactured by Kolon Life Science Inc.) (0.1 wt. % Tween 80)
Negative control 2 (blank): (I and II) 0.1 wt. % aqueous solution of Tween 80

**[Table 6]**

| Hair weight (mg) | | | | | | |
|---|---|---|---|---|---|---|
| Animal No. | Sample 2-1 | | Sample 2-2 | | Negative control 2 | |
| | I | II | I | II | I | II |
| 1 | 23.19 | 21.50 | 19.01 | 22.52 | 20.08 | 15.90 |
| 2 | 24.72 | 16.44 | 18.44 | 26.56 | 16.31 | 22.75 |
| 3 | 20.41 | 25.08 | 23.89 | 18.15 | 20.54 | 15.03 |
| Average value | 21.89 | | 21.43 | | 18.44 | |
| Standard deviation | 3.22 | | 3.44 | | 3.11 | |
| Standard error | 1.31 | | 1.40 | | 1.27 | |
| Rate of hair increase vs. negative control | 19% | | 16% | | - | |

### Comparative Example 5

A hair restoration test was carried out using six male 7-week-old Hartley guinea pigs in the same manner as in Comparative Example 4. The results are shown in Table 7.
Sample 3-1: (I and II) aqueous suspension of 2.0 wt. % of 48% aqueous dispersion liquid of zinc pyrithione ("CleanBio-Zinc", manufactured by Kolon Life Science Inc.) + 1.5 wt. % ultrafine zinc oxide ("MZ-300", manufactured by Tayca Corporation, average particle diameter: 0.03 µm) (0.1 wt. % Tween 80)
Sample 3-2: (I and II) 1.50 wt. % aqueous suspension of ultrafine zinc oxide ("MZ-300", manufactured by Tayca Corporation, average particle diameter: 0.03 µm) (0.1 wt. % Tween 80)
Negative control 3 (blank): (I and II) 0.1 wt. % aqueous solution of Tween 80

**[Table 7]**

| Hair weight (mg) | | | | | | |
|---|---|---|---|---|---|---|
| Animal No. | Sample 3-1 | | Sample 3-2 | | Negative control 3 | |
| | I | II | I | II | I | II |
| 1 | 30.09 | 25.86 | 19.23 | 23.20 | 21.31 | 16.60 |
| 2 | 21.29 | 16.65 | 20.63 | 15.94 | 14.82 | 23.45 |
| 3 | 19.93 | 26.76 | 17.15 | 21.91 | 18.38 | 17.56 |
| Average value | 23.43 | | 19.68 | | 18.69 | |
| Standard deviation | 4.98 | | 2.79 | | 3.17 | |
| Standard error | 2.03 | | 1.14 | | 1.29 | |
| Rate of hair increase vs. negative control | 25% | | 5% | | - | |

Based on the results of Example 9 (Table 5) and Comparative Examples 4 and 5 (Tables 6 and 7), the composites of the present invention (Samples 1-1 and 1-2) exhibited clearly superior hair restoration effects in comparison with any of the mixture of the complex compound and zinc oxide produced as a by-product (Sample 2-1), zinc pyrithione (Sample 2-2), the composition of zinc pyrithione and ultrafine zinc oxide (Sample 3-1), and ultrafine zinc oxide (Sample 3-2).

### Example 10

Conversion speeds of the composite of a zinc pyrithione/zinc oxide complex compound and ultrafine zinc oxide as well as zinc pyrithione into copper pyrithione under the action of an aqueous copper sulfate solution were visually determined. The results are shown in Table 8.
Sample 1: 5 wt. % aqueous suspension of white powder of Example 3
Sample 2: 5 wt. % aqueous suspension of zinc pyrithione powder (manufactured by API Corporation)
Sample 3: 5 wt. % aqueous suspension containing equal amounts of white powder of Example 3 and zinc pyrithione powder (manufactured by API Corporation)
Test method: In 100 mL of water, 400 mg of copper sulfate heptahydrate was dissolved so as to prepare an aqueous copper sulfate solution having a molar concentration of approximately one-tenth of that of the sample. Into test tubes having 2 mL of the samples, 20 mL of the aqueous copper sulfate solution was added, followed by shaking. Time courses of complete formation of moss-green precipitates unique to copper pyrithione were observed (water temperature: 15°C).

**[Table 8]**

| Time course of complete conversion into copper pyrithione | | | |
|---|---|---|---|
| | After 3 minutes | After 10 minutes | After 30 minutes |
| Sample 1 | No coloring | Slightly colored to yellow | Colored to moss-green |
| Sample 2 | Colored to moss-green | Moss- green | Moss- green |
| Sample 3 | Colored to moss-green | Moss- green | Moss- green |

From the above table, it is found that the composite of a zinc pyrithione/zinc oxide complex compound and ultrafine zinc oxide is more resistant to a metal substitution reaction with copper ions compared to zinc pyrithione.

### Example 11

Formulation example of a shampoo containing the composite of a complex compound and ultrafine zinc oxide
A liquid shampoo was prepared by homogenously mixing each component of the following composition.

| | |
|---|---|
| sodium lauryl sulfate | 16 wt. % |
| white powder of Example 1 | 0.5 wt. % |
| hydroxyethyl cellulose | 0.3 wt. % |
| citric acid | trace |
| purified water | balance |
| Total | 100.0 wt. % |

### Example 12

Formulation example of a shampoo containing the composite of a complex compound and ultrafine zinc oxide as well as ultrafine zinc oxide
A liquid shampoo was prepared by homogenously mixing each component of the following composition.

| | |
|---|---|
| polyoxyethylene (EO = 2 mol) sodium lauryl sulfate | 16.0 wt. % |
| white powder of Example 3 | 1.0 wt. % |
| ultrafine zinc oxide (manufactured by Tayca Corporation, average | |
| particle diameter: 0.03 µm) | 1.5 wt. % |
| propylene glycol | 0.3 wt. % |
| citric acid | trace |
| purified water | balance |
| Total | 100.0 wt. % |

### Example 13

Formulation example of a shampoo containing the composite of a complex compound and ultrafine zinc oxide as well as miconazole nitrate
A liquid shampoo was prepared by homogenously mixing each component of the following composition.

| | |
|---|---|
| polyoxyethylene (EO = 2 mol) monoethanolamine lauryl ether sulfate | 16.0 wt. % |
| white powder of Example 4 (B) | 0.3 wt. % |
| miconazole nitrate (reagent manufactured by Sigma-Aldrich Corporation) | 0:5 wt. % |
| propylene glycol | 0.3 wt. % |
| citric acid | trace |
| purified water | balance |
| Total | 100.0 wt. % |

### Example 14

Formulation example of a shampoo containing the composite of zinc pyrithione and ultrafine zinc oxide
A liquid shampoo was prepared by homogenously mixing each component of the following composition.

| | |
|---|---|
| monoethanolamine lauryl sulfate | 16.0 wt. % |
| white powder of Example 2 | 0.8 wt. % |
| hydroxyethyl cellulose | 0.3 wt. % |
| citric acid | trace |
| purified water | balance |
| Total | 100.0 wt. % |

### Example 15

Formulation example of a ship-bottom paint containing the composite of a complex compound and ultrafine zinc oxide
A ship-bottom paint was prepared by homogenously mixing each component of the following composition using a propeller pulverizer.
Copolymer of methylmethacrylate and triisopropylsilyl acrylate (2:3) (50% xylene solution)

| | |
|---|---|
| cuprous oxide | 36 wt. % 35 wt. % |
| white powder of Example 3 | 5 wt. % |
| zinc oxide | 5 wt. % |
| titanium white | 1 wt. % |
| red iron oxide | 1 wt. % |
| fatty acid amide wax (20%) | 2 wt. % |
| xylene | 15 wt. % |
| Total | 100 wt. % |

### Example 16

Formulation example of an antifouling agent for a fish-farming net containing the composite of a complex compound and ultrafine zinc oxide
An antifouling agent for a fish-farming net was prepared by homogenously mixing each component of the following composition.

| | |
|---|---|
| copolymer of butylacrylate and methylmethacrylate (50% xylene solution) | 20 wt. % |
| pyridine triphenylborane | 5wt.% |
| white powder of Example 1 | 6 wt. % |
| polyether silicone oil | 2wt.% |
| Disparlon 4200-20 (manufactured by Kusumoto Chemicals, Ltd.) | 3 wt. % |
| xylene | 64 wt. % |
| Total | 100 wt. % |

### Example 17

A preparation of an antiseptic and anti-mold agent containing the composite of zinc pyrithione and ultrafine zinc oxide
An antiseptic and anti-mold aqueous suspension preparation was prepared by homogenously mixing each component of the following composition and adjusting the mixture at pH 7.

| | |
|---|---|
| white powder of Example 2 | 6.0 wt. % |
| fine amorphous silicon oxide (Aerosil 200) | 1.0 wt. % |
| sodium carboxymethylcellulose | 0.1 wt. % |
| water | balance |
| Total | 100.0 wt. % |

### Example 18

A preparation of the composite of zinc pyrithione and ultrafine zinc oxide + an isothiazolone antiseptic and anti-mold agent
An antiseptic and anti-mold aqueous suspension preparation was prepared by homogenously mixing each component of the following composition and adjusting the mixture at pH 8.

| | |
|---|---|
| zinc pyrithione | 4.0 wt. % |
| ultrafine zinc oxide (average particle diameter: 0.03 µm) | 1.0 wt. % |
| 50% aqueous solution of 2-methyl-4-isothiazolin-3-one | 4.0 wt. % |
| bentonite | 1.0 wt. % |
| sodium carboxymethylcellulose | 0.1 wt. % |
| sodium hydroxide | trace |
| water | balance |
| Total | 100.0 wt. % |

### INDUSTRIAL APPLICABILITY

The physiologic/antibiotic active composite of the present invention containing zinc pyrithione or a zinc pyrithione/zinc oxide complex compound and ultrafine zinc oxide having an average particle diameter of 0.01 to 0.15 µm has a superior antidandruff and hair restoration effect, antimicrobial activity, and resistance to copper ions in comparison with commercially available zinc pyrithione. Thus, the physiologic/antibiotic active composite of the present invention has industrial applicability as an active component to be used for an antidandruff agent and a hair restoration-promoting agent for a shampoo, an antifouling agent for a ship-bottom paint, and an antiseptic and anti-mold agent for industrial products.

## Claims

1. A new crystalline zinc pyrithione/zinc oxide composite obtained by treating an aqueous suspension or an aqueous paste comprising zinc pyrithione or zinc pyrithione/ amorphous zinc oxide complex compound and ultrafine zinc oxide having an average particle diameter of 0.01 to 0.15 µm at pH 8 to 12, the zinc pyrithione or the zinc pyrithione/ amorphous zinc oxide complex compound being represented by the general formula (I):
xZnO·ZnPy₂ (I)
wherein, x represents 0 or a positive number satisfying 0≤x≤1 and Py represents a 2-pyridylthio-N-oxide group.

2. The new crystalline zinc pyrithione/zinc oxide composite according to claim 1, which is obtained by treating the aqueous suspension or the aqueous paste at pH 8 to 9.

3. The new crystalline zinc pyrithione/zinc oxide composite according to claim 1 or 2, wherein an amount of the ultrafine zinc oxide having an average particle diameter of 0.01 to 0.15 µm is 1 to 50 parts by weight per 100 parts by weight of the zinc pyrithione or the zinc pyrithione/zinc oxide complex compound represented by the general formula (I) according to claim 1 in the aqueous suspension or the aqueous paste.

4. A physiologic/antibiotic active composition comprising the new crystalline zinc pyrithione/zinc oxide composite according to any of claims 1 to 3.

5. The physiologic/antibiotic active composition according to claim 4, which is an antidandruff and hair restoration agent.

6. The physiologic/antibiotic active composition according to claim 5, further comprising an azole antifungal agent.

7. The physiologic/antibiotic active composition according to claim 5 or 6, which is a shampoo.

8. The physiologic/antibiotic active composition according to claim 4, which is an underwater antifouling agent.

9. The physiologic/antibiotic active composition according to claim 4 or 8, which is a ship-bottom paint further comprising cuprous oxide.

10. The physiologic/antibiotic active composition according to claim 4, which is an antiseptic and anti-mold agent.

11. The physiologic/antibiotic active composition according to claim 10, which is an aqueous antiseptic and anti-mold suspension further comprising an isothiazolone antiseptic and anti-mold agent.
